# EUROPÄISCHE PATENTANMELDUNG

(11) **EP 3 473 227 A1**
(43) Veröffentlichungstag der Anmeldung: **24.04.2019**
(21) Anmeldenummer: 17196952.0
(22) Anmeldetag: 17.10.2017
(51) Int. Cl.: A61F 15/00, B65D 85/67, B65H 18/10

(54) **VORRICHTUNGEN ZUR HANDHABUNG AN EINEN VERWENDUNGSORT ANZULEGENDER MATERIALBAHNEN**

(71) Anmelder: Klinikum rechts der Isar der Technischen Universität München, 81675 München (DE)
(72) Erfinder: Zink, Alexander, 80802 München (DE); Zink, Alexander, 86510 Ried (DE)
(74) Vertreter: Koplin, Moritz

(57) **Zusammenfassung**

Gezeigt werden Vorrichtungen mit einem Gehäuse und einem in dem Gehäuse drehbar gelagerten Element einer Wickeleinheit, welches zum Einziehen einer Materialbahn, vorzugweise einer medizinischen Binde, in das Gehäuse und zum Aufwickeln der Materialbahn eingerichtet ist.

## Beschreibung

### GEBIET

Die vorliegende Erfindung bezieht sich auf Vorrichtungen zur Handhabung an einen Verwendungsort anzulegender Materialbahnen. Insbesondere bezieht sich die vorliegende Erfindung auf Vorrichtungen zur Handhabung medizinischer Binden.

### HINTERGRUND

Medizinische Binden werden beim Anlegen oftmals manuell von einer Rolle abgewickelt, wobei die Spannung, mit der die Binde beim Anlegen beaufschlagt wird, durch den Benutzer individuell vorgegeben wird. Soll eine benutzte Binde wiederverwendet werden, wird die abgenommene Binde typischerweise gereinigt/desinfiziert und in Vorbereitung des erneuten Anlegens manuell aufgewickelt. Dadurch ist die Handhabung medizinischer Binden oftmals mit erheblichem Aufwand verbunden, der einen wirtschaftlichen Einsatz derselben erschwert.

### ZUSAMMENFASSUNG

Die Erfindung bereichert diesbezüglich den Stand der Technik, als Vorrichtungen bereitgestellt werden, die den Benutzer beim Anlegen, aber auch beim Wiederverwenden von medizinischen Binden unterstützen. Die Erfindung ist zudem nicht auf die Handhabung medizinischer Binden beschränkt. Vielmehr können die erfindungsgemäßen Vorrichtungen zur Handhabung beliebiger aufwickelbarer und an einen Verwendungsort anzulegender Materialbahnen verwendet werden.

Eine erste erfindungsgemäße Vorrichtung, zur Handhabung von an einen Verwendungsort anzulegenden bandförmigen Materialbahnen, vorzugsweise Binden, umfasst ein Gehäuse mit einem in dem Gehäuse drehbar gelagerten Element einer Wickeleinheit, welche zum Einziehen einer Materialbahn in das Gehäuse und zum Aufwickeln der Materialbahn eingerichtet ist, und eine der Wickeleinheit vor- oder nachschaltbare Zuführeinheit zum Aufbringen eines in einem Vorratsbehälter bevorrateten festen, flüssigen oder gasförmigen Stoffes auf die Materialbahn.

Dabei sind unter dem Begriff "Handhabung", wie er in der Beschreibung und den Ansprüchen verwendet wird, insbesondere Handlungen zu verstehen, die der Vorbereitung des Anlegens bzw. dem Anlegen selbst dienen. Ferner ist unter dem Begriff "bandförmig", wie er in der Beschreibung und den Ansprüchen verwendet wird, insbesondere eine flache, längliche und flexible Struktur gleichbleibender Breite und (im Wesentlichen) gleichbleibender Dicke zu verstehen. Des Weiteren ist unter dem Begriff "Verwendungsort", wie er in der Beschreibung und den Ansprüchen verwendet wird, insbesondere ein Oberflächenbereich zu verstehen, an dem die Materialbahn (nach dem Anlegen) anliegt.

Zudem ist unter dem Begriff "Materialbahn", wie er in der Beschreibung und den Ansprüchen verwendet wird, insbesondere ein in eine flache und längliche Form gebrachtes Material zu verstehen, wobei eine Zugbelastung unterhalb einer bestimmten Schwelle eine elastische (im Wesentlichen reversible) Dehnung des Materials hervorruft. Ferner ist unter dem Begriff "Binde", wie er in der Beschreibung und den Ansprüchen verwendet wird, insbesondere eine Fasern-aufweisende, bandförmige Materialbahn (bspw. eine bandförmige Gewebe- oder Vliesbahn) zu verstehen.

Ferner versteht es sich, dass die erste erfindungsgemäße Vorrichtung zur Aufnahme einer einzelnen Materialbahn, aber auch zur Aufnahme einer Vielzahl an Materialbahnen eingerichtet sein kann. Dabei können neben typgleichen Materialbahnen auch Materialbahnen unterschiedlichen Typs nebeneinander oder übereinander aufgewickelt werden. Bspw. können bei mehreren und insbesondere bei verschiedenen nacheinander anzulegenden Materialbahnen diese in umgekehrter Reihenfolge übereinander aufgewickelt werden.

Die der Wickeleinheit vor- oder nachschaltbare Zuführeinheit vereinfacht dabei das Aufbringen von festen, flüssigen oder gasförmigen Stoffen (bspw. desinfizierenden und/oder heilungsfördernden Stoffen, wie Epigallocatechingallat, EGCG) auf die Materialbahnen und erleichtert so deren Handhabung, da ein manuelles Aufbringen als separater Schritt vor dem Aufwickeln bzw. nach dem Abwickeln entfällt.

Vorzugsweise ist das Gehäuse von der der Wickeleinheit vor- oder nachschaltbaren Zuführeinheit abkoppelbar und besonders vorzugsweise werkzeuglos abkoppelbar und weist besonders vorzugsweise eine spaltförmige Öffnung zum Hindurchführen der Materialbahn auf.

Bspw. kann das Gehäuse der Wickeleinheit mit einem Gehäuse der Zuführeinheit form- oder kraftschlüssig verbindbar sein, so dass sich die Gehäuse in angekoppeltem Zustand in einer bestimmten Relativlage zueinander befinden. Die bestimmte Relativlage kann z. B. so gewählt sein, dass sich die spaltförmige Öffnung des Gehäuses der Wickeleinheit und eine spaltförmige Öffnung des Gehäuses der Zuführeinheit gegenüberliegen und vorzugsweise miteinander fluchten.

Die Wickeleinheit kann dadurch separat von der Zuführeinheit betrieben werden. Dies kann wiederum das Anlegen der Materialbahnen erleichtern, da die Wickeleinheit naturgemäß kompakter und leichter ist, als die gesamte Vorrichtung. Zudem kann die Zuführeinheit in Kombination mit einer Vielzahl von Wickeleinheiten verwendet werden, die zum Aufwickeln von Materialbahnen an die Zuführeinheit angekoppelt werden, wobei die Materialbahnen nach dem Abkoppeln entweder direkt einer Verwendung zugeführt oder (bspw. im Gehäuse der Wickeleinheit) gelagert werden können.

Vorzugsweise ist die Wickeleinheit zum Abwickeln der Materialbahn eingerichtet, und stellt eine, einer beim Abwickeln aufzuwendenden Zugkraft entgegenwirkende, einstellbare Rückhaltekraft, Bremskraft, oder Rückstellkraft bereit.

Eine einstellbare Rückhaltekraft/Bremskraft kann z. B. durch eine mittels einer (bspw. in ihrer Höhe einstellbaren) spaltförmigen Öffnung des Gehäuses auf die Materialbahn ausgeübten Reibkraft (Klemmkraft) oder eine auf das drehbar gelagerte Element wirkende einstellbare Reibkraft (Klemmkraft) bewirkt werden. Die Rückstellkraft kann bspw. durch eine mit dem drehbar gelagerten Element gekoppelte Konstantkraftfeder bewirkt werden. Durch die einstellbare Rückhaltekraft/Bremskraft bzw. die Rückstellkraft kann die Spannung, mit der die Materialbahn beim Anlegen beaufschlagt wird, eingestellt bzw. konstant gehalten werden. Dadurch kann typischerweise eine gleichmäßigere Materialbahnspannung erzielt werden, als bei einem rein manuellen Anlegen. Zudem kann die Gefahr eines unkontrollierten Abwickelns der Materialbahn reduziert werden, wenn bspw. das Gehäuse der Wickeleinheit einem Benutzer derselben beim Anlegen der Materialbahn aus der Hand rutscht.

Vorzugsweise ist die der Wickelvorrichtung vor- oder nachschaltbare Zuführeinheit eingerichtet, den in dem Vorratsbehälter bevorrateten festen, flüssigen oder gasförmigen Stoff beim Einziehen der Materialbahn in das Gehäuse auf die Materialbahn aufzubringen.

Dies reduziert die Komplexität der Vorrichtung, da dadurch die aus dem Schritt des Anlegens der Materialbahn resultierenden Vorgaben/Beschränkungen hinsichtlich der Kontinuität und Geschwindigkeit der Materialbahn (beim Abwickeln), beim Aufbringen des Stoffes nicht berücksichtigt werden müssen.

Vorzugsweise ist die Vorrichtung eingerichtet, den Massestrom des beim Einziehen der Materialbahn in das Gehäuse auf die Materialbahn aufgebrachten festen, flüssigen oder gasförmigen Stoffes in Abhängigkeit von der Einziehgeschwindigkeit zu steuern, wobei der Massestrom versiegt, wenn die Einziehgeschwindigkeit null ist.

Dadurch kann erreicht werden, dass der aufgebrachte Stoff möglichst gleichmäßig über die Materialbahn verteilt wird.

Vorzugsweise ist ein Minimalwert der Einziehgeschwindigkeit einstellbar oder ist die Einziehgeschwindigkeit auf einen konstanten Wert einstellbar.

Bspw. kann die Einziehgeschwindigkeit an der Vorrichtung mechanisch oder elektronisch eingestellt werden, z. B. durch Steuern eines mit einer Primär- oder Sekundärzelle gespeisten Elektromotors.

Vorzugsweise weist die Wickeleinheit eine Energiespeichereinheit auf, besonders vorzugsweise eine Konstantkraftfeder, der durch Drehen des drehbar gelagerten Elements in eine Richtung Energie zugeführt und durch Drehen des drehbar gelagerten Elements in die andere Richtung Energie entnommen werden kann.

Dadurch kann der durch das Austauschen/Wideraufladen der Primär- oder Sekundärzellen entstehende Aufwand vermieden werden.

Vorzugsweise umfasst die vor- oder nachschaltbare Zuführeinheit eine Spanneinrichtung, besonders vorzugsweise eine Spannrolle, die dazu eingerichtet ist, einen zwischen der Spanneinrichtung und dem im Gehäuse drehbar gelagerten Element geführten Materialbahnabschnitt beim Einziehen der Materialbahn zu spannen.

Dadurch kann ein (weitgehend) faltenfreies Aufwickeln der Materialbahn erreicht werden.

Vorzugsweise ist die Wickeleinheit eingerichtet, eine die Drehung des in dem Gehäuse drehbar gelagerten Elements verhindernde Sperrung aufzuheben, wenn auf einen durch das Gehäuse geführten und an dem Element befestigten Materialbahnabschnitt eine Zugspannung ausgeübt wird.

Dadurch kann ein unbeabsichtigtes Abwickeln der Materialbahn verhindert werden.

Vorzugsweise umfasst die vor- oder nachschaltbare Zuführeinheit eine Rolle, über die der Materialbahnabschnitt geführt wird, und ist die vor- oder nachschaltbare Zuführeinheit eingerichtet, den festen, flüssigen oder gasförmigen Stoff mittels der Rolle auf den Materialbahnabschnitt aufzubringen.

Dadurch kann zu Zuführung von Stoffen beim Aufbringen auf die Materialbahn vereinfacht werden.

Vorzugsweise weist die vor- oder nachschaltbare Zuführeinheit eine an den Vorratsbehälter angeschlossene Düse auf, die eingerichtet ist, den festen, flüssigen oder gasförmigen Stoff direkt oder indirekt auf den Materialbahnabschnitt aufzubringen.

Bspw. kann die Düse einen festen oder flüssigen Stoff zerstäuben/versprühen, wobei das dabei entstehende Aerosol auf die Rolle (und von der Rolle bspw. mittels Adhäsion auf den über die Rolle geführten Materialabschnitt) oder direkt auf den Materialabschnitt befördert werden kann.

Vorzugsweise ist der Vorratsbehälter in der Rolle angeordnet oder an die Rolle angeschlossen und ist die vor- oder nachschaltbare Zuführeinheit eingerichtet, den in dem Vorratsbehälter bevorrateten festen, flüssigen oder gasförmigen Stoff über Öffnungen oder Aussparungen in der Rollenoberfläche auf den Materialbahnabschnitt aufzubringen.

Bspw. kann ein flüssiger Stoff mittels Kapillarkraft zur Rollenoberfläche befördert und bspw. mittels Adhäsion von der Materialbahn aufgenommen werden.

Eine zweite erfindungsgemäße Vorrichtung, zur Handhabung von Binden, umfasst ein Gehäuse mit einem in dem Gehäuse drehbar gelagerten Element einer Wickeleinheit, welches zum Einziehen einer Materialbahn in das Gehäuse und zum Aufwickeln der Materialbahn eingerichtet ist, wobei die Wickeleinheit eingerichtet ist, eine die Drehung des in dem Gehäuse drehbar gelagerten Elements verhindernde Sperrung aufzuheben, wenn auf einen durch das Gehäuse geführten und an dem Element befestigten Materialbahnabschnitt eine Zugspannung ausgeübt wird, die oberhalb einer bestimmten Zugspannungsschwelle liegt.

Dadurch kann, wie oben angeführt, ein unbeabsichtigtes Abwickeln und insbesondere ein Verschmutzen medizinischer Binden beim ungewollten Abwickeln der Binde vermieden werden.

Vorzugsweise weist die Wickeleinheit eine Energiespeichereinheit, besonders vorzugsweise eine Konstantkraftfeder auf, der durch Drehen des drehbar gelagerten Elements in eine Richtung Energie zugeführt und durch Drehen des drehbar gelagerten Elements in die andere Richtung Energie entnommen werden kann.

Dadurch kann, wie oben angeführt, der durch das Austauschen/Wideraufladen der Primär- oder Sekundärzellen entstehende Aufwand vermieden werden.

Ein erfindungsgemäßes Verfahren zum Vorbereiten und Anlegen von Binden umfasst ein Einziehen einer ersten Binde in das Gehäuse einer erfindungsgemäßen Vorrichtung und ein Aufwickeln der ersten Binde, ein Abwickeln und Anlegen der ersten Binde, ein Einziehen einer zweiten Binde in das Gehäuse der Vorrichtung und ein Aufwickeln der zweiten Binde und ein Abwickeln und Anlegen der zweiten Binde, wobei beim Einziehen der Binden vorzugsweise ein Desinfektions- und/oder ein heilförderndes Mittel auf die Binde aufgebracht wird.

Dabei können, wie oben angeführt, in einem Aufwickelschritt (ohne dazwischenliegendem Abwickelschritt) mehrere Binden nacheinander aufgewickelt werden, so dass in dem Gehäuse mehrere Binden übereinander oder nebeneinander aufgewickelt sind. Bspw. kann das drehbare Element zwei Abschnitte aufweisen, die entkoppelt werden können, so dass die entkoppelten Abschnitte relativ zueinander gedreht werden können, wodurch ein gleichzeitiges Aufwickeln (im gekoppelten Zustand) und ein getrenntes Abwickeln (im entkoppelten Zustand) möglich ist.

Zudem kann ein Element zum Befestigen am Ende einer aufzuwickelnden Materialbahn/Binde bereitgestellt werden, welches so dimensioniert ist, dass es beim Einziehen der Materialbahn/Binde an der Gehäuseöffnung "festgehalten" wird und dadurch den Einziehvorgang stoppt. Dadurch kann das Einziehen weiter automatisiert werden und bedarf nur einer eingeschränkten Überwachung.

Ferner versteht es sich, dass alle Merkmale (bevorzugter Ausführungsformen) der erfindungsgemäßen Vorrichtungen auch Merkmale (bevorzugter Ausführungsformen) des Verfahrens sein können, welches sich auf die Verwendung der erfindungsgemäßen Vorrichtungen bezieht, und umgekehrt.

### KURZBESCHREIBUNG DER ZEICHNUNGEN

Die Erfindung wird nachfolgend in der detaillierten Beschreibung anhand von Ausführungsbeispielen erläutert, wobei auf Zeichnungen Bezug genommen wird, in denen:
Fig. 1a und 1b beispielhafte schematische Darstellungen einer erfindungsgemäßen Vorrichtung beim Auf- und Abwickeln einer Materialbahn zeigen; Fig. 2a, 2b, 2c und 2d beispielhafte schematische Darstellungen erfindungsgemäßer Vorrichtungen beim Auf- und Abwickeln einer Materialbahn zeigen;
Fig. 3a, 3b und 3c beispielhafte schematische Darstellungen der Wickeleinheit zeigen;
Fig. 4a, 4b und 4c beispielhafte schematische Darstellungen der Zuführeinheit zeigen;
Fig. 5, 5a, und 5b beispielhafte schematische Darstellungen des Vorratsbehälters zeigen;
Fig. 6a, 6b, 7a und 7b Schnittansichten beispielhafter Ausgestaltungen erfindungsgemäßer Vorrichtungen zeigen;
Fig. 8 eine perspektivische Ansicht des Gehäuses der Wickeleinheit in Fig. 7a und 7b zeigt;
Fig. 9 eine Modifikation der in Fig. 6a, 6b, 7a und 7b gezeigten beispielhaften Ausgestaltungen illustriert; und
Fig. 10 ein Ablaufdiagramm eines Verfahrens zum Vorbereiten und Anlegen von Binden zeigt.

Dabei sind in den Zeichnungen gleiche und funktional ähnliche Elemente durch gleiche Bezugszeichen gekennzeichnet. Jedoch versteht es sich, dass nicht notwendigerweise alle Elemente in allen Zeichnungen gezeigt sind und dass die gezeigten Elemente nicht notwendigerweise maßstabsgetreu dargestellt sind.

### DETAILLIERTE BESCHREIBUNG

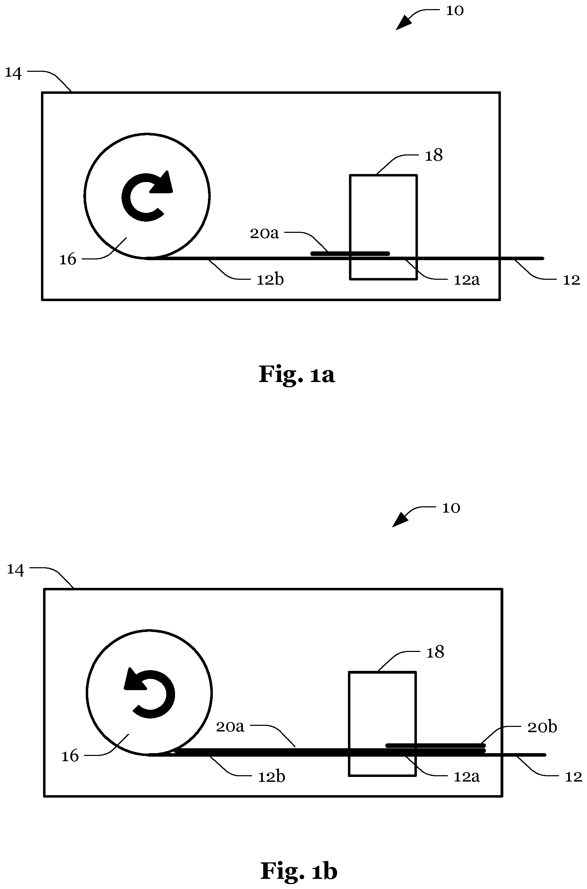

Fig. 1a zeigt eine schematische Darstellung einer beispielhaften Ausgestaltung einer Vorrichtung 10 zur Handhabung bandförmiger Materialbahnen 12 wie bspw. (medizinischer) Binden. Die Vorrichtung 10 umfasst ein (luftdicht verschließbares) Gehäuse 14 mit einem in dem Gehäuse 14 drehbar gelagerten Element einer Wickeleinheit 16 (bspw. einem Wickeldorn oder einer Wickelrolle). Die Wickeleinheit 16 ist zum Einziehen der Materialbahn 12 in das Gehäuse 14 und zum Aufwickeln der Materialbahn 12 eingerichtet. Bspw. kann die Wickeleinheit 16 einen elektrischen Motor, eine Konstantkraftfeder 90 oder eine mechanische Betätigungseinrichtung, wie z. B. eine Kurbel 36, 102, umfassen, durch den bzw. die das in dem Gehäuse 14 drehbar gelagerte Element angetrieben werden kann.

Wird das in dem Gehäuse 14 drehbar gelagerte Element angetrieben, kann eine an dem drehbar gelagerten Element befestigte bzw. eine um das drehbar gelagerte Element geschlungene Materialbahn 12 um das drehbar gelagerte Element aufgewickelt werden. Um die Materialbahn 12 an dem drehbar gelagerten Element befestigen bzw. um das drehbar gelagerte Element schlingen zu können, kann das Gehäuse 14 zweiteilig ausgeführt sein, wobei durch Öffnen (d. h. Abnehmen oder Wegklappen) eines Gehäusedeckels ein Zugang zum drehbar gelagerten Element geschaffen werden kann, welcher ein Einfädeln der Materialbahn 12 ermöglicht.

Wie in Fig. 1a gezeigt, kann in dem Gehäuse 14 eine Zuführeinheit 18 angeordnet sein, welche zum Aufbringen eines festen, flüssigen oder gasförmigen Stoffes 20a auf die Materialbahn 12 eingerichtet ist. Bspw. kann die Zuführeinheit 18 entlang eines Pfades der Materialbahn 12 angeordnet sein, wobei der feste, flüssige oder gasförmige Stoff 20a auf den die Zuführeinheit 18 passierenden Materialbahnabschnitt 12a aufgebracht werden kann. Der die Zuführeinheit 18 passierende Materialbahnabschnitt 12a kann dabei mittels einer, auf den an der Wickeleinheit 16 befestigten Materialbahnabschnitt 12b ausgeübten Zugspannung durch die Zuführeinheit 18 gezogen werden.

Wie in Fig. 1b gezeigt, kann auch beim Abwickeln der Materialbahn 12, bei dem der an der Wickeleinheit 16 befestigte Materialbahnabschnitt 12b mit einer Zugspannung in umgekehrter Richtung beaufschlagt wird, ein fester, flüssiger oder gasförmiger Stoff 20b auf die Materialbahn 12 aufgebracht werden. Bspw. kann ein erster fester, flüssiger oder gasförmiger Stoff 20a während des Aufwickelns der Materialbahn 12 auf die Materialbahn 12 aufgebracht werden und ein zweiter fester, flüssiger oder gasförmiger Stoff 20b während des Abwickelns der Materialbahn 12 auf die Materialbahn 12 aufgebracht werden. Dies kann z. B. dann vorteilhaft sein, wenn der erste feste, flüssige oder gasförmige Stoff 20a mit dem zweiten festen, flüssigen oder gasförmigen Stoff 20b unmittelbar vor dem Anlegen reagieren soll, der erste feste, flüssige oder gasförmige Stoff 20a einer bestimmten Vorlaufs- oder Einwirkzeit (vor dem Anlegen) bedarf, der zweite feste, flüssige oder gasförmige Stoff 20b flüchtig ist, etc.

Allerdings versteht es sich, dass das Aufbringen fester, flüssiger oder gasförmiger Stoffe 20a, 20b auf die Materialbahn 12 anstatt während des Aufwickelns und während des Abwickelns der Materialbahn 12 auch nur während des Aufwickelns der Materialbahn 12 oder während des Abwickelns der Materialbahn 12 erfolgen kann. Ferner kann das Aufbringen fester, flüssiger oder gasförmiger Stoffe 20a, 20b auf die Materialbahn 12 in bestimmten Situationen auch gänzlich unterbleiben. Des Weiteren kann die Zuführeinheit 18 eingerichtet sein, während des Aufwickelns und/oder während des Abwickelns der Materialbahn 12 anstatt eines einzigen festen, flüssigen oder gasförmigen Stoffes 20a, 20b mehrere feste, flüssige oder gasförmige Stoffe 20a, 20b gleichzeitig auf den die Zuführeinheit 18 passierenden Materialbahnabschnitt 12a aufzubringen.

Zudem kann an der Zuführeinheit 18 einstellbar sein, ob ein fester, flüssiger oder gasförmiger Stoff 20a, 20b über die volle Breite der Materialbahn 12 oder nur einen Teil der Materialbahnbreite aufzubringen ist, bspw. über ein oder mehrere (teilweise) verschließbare oder schwenkbare Abgabeelemente (z. B. Düsen 42) der Zuführeinheit 18. Ferner kann anstatt eines (in Längsrichtung) gleichmäßigen Auftragens eines festen, flüssigen oder gasförmigen Stoffes 20a, 20b auch ein in Längsrichtung abschnittsweises Auftragen eines festen, flüssigen oder gasförmigen Stoffes 20a, 20b erfolgen. Des Weiteren kann ein abschnittsweises, evtl. in Längs- und/oder Querrichtung zueinander versetztes Auftragen der festen, flüssigen oder gasförmigen Stoffe 20a, 20b erfolgen, so dass feste, flüssige oder gasförmige Stoffe 20a, 20b in Längsrichtung (nicht oder nicht nur überdeckend, sondern) nebeneinander oder aufeinander folgend aufgebracht werden.

Bspw. kann die Zuführeinheit 18 (bzw. mittelbar der durch die Zuführeinheit auf die Materialbahn 12 aufgebrachte Stoffstrom) mit der Betätigung oder dem Antrieb der Wickeleinheit 16 (mechanisch oder signaltechnisch) gekoppelt sein, die Zuführeinheit 18 (bzw. mittelbar der durch die Zuführeinheit auf die Materialbahn 12 aufgebrachte Stoffstrom) mit der Bewegung der Materialbahn 12 (unter Verwendung mechanischer Bauteile wie z. B. Zahnräder oder Riemen) synchronisiert sein, oder die Zuführeinheit 18 mit einem Sensor (nicht gezeigt) zum Erfassen der Bewegung/Geschwindigkeit der Materialbahn 12 versehen bzw. gekoppelt sein. Diese Maßnahmen können ferner dazu genutzt werden, die Zuführeinheit 18 dazu auszubilden, den Stoffstrom auf einen bestimmten Wert (bspw. null oder im Wesentlichen null) zu reduzieren, wenn die Materialbahn 12 (während Aufwickelpausen oder Abwickelpausen) nicht bewegt wird, indem bspw. eine Pumpgeschwindigkeit auf Basis mechanischer und/oder signaltechnischer Mittel an die Materialbahngeschwindigkeit angepasst wird.

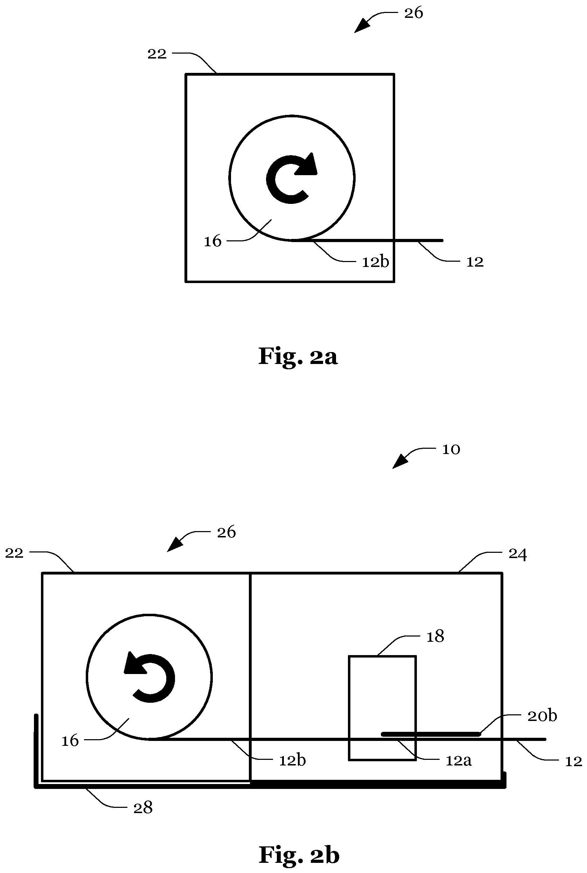

Fig. 2a und 2b zeigen eine schematische Darstellung einer Abwandlung der beispielhaften Ausgestaltung der Vorrichtung 10 zur Handhabung bandförmiger Materialbahnen 12, wie bspw. medizinischer Binden, welche sich von der in Fig. 1a und 1b gezeigten Vorrichtung 10 dadurch unterscheidet, dass das (luftdicht verschließbare) Gehäuse 22, in dem das drehbar gelagerte Element der Wickeleinheit 16 angeordnet ist, von dem Gehäuse 24 der Zuführeinheit 18 mechanisch abkoppelbar ist. Das Abkoppeln kann bspw. durch Lösen eines Verschlusses (mittels eines Werkzeuges oder manuell) oder durch (werkzeugloses) Abziehen des Gehäuses 22 der Wickeleinheit 16 von dem Gehäuse 24 der Zuführeinheit 18 erfolgen. Analog kann das Ankoppeln des Gehäuses 22 der Wickeleinheit 16 an das Gehäuse 24 der Zuführeinheit 18 durch ein (werkzeugloses) Aufstecken/Aufrasten bzw. das Schließen eines Verschlusses (mittels eines Werkzeuges oder manuell) erfolgen.

Wie in Fig. 2a und 2b gezeigt, können die Gehäuse 22, 24 jeweils spaltförmige Öffnungen zum Einziehen/Herausziehen der Materialbahn 12 aufweisen, die in angekoppeltem Zustand sich gegenüberliegen und miteinander (in Längsrichtung der Materialbahn 12) fluchten. Dabei können zur Vereinfachung des Einfädelns der Materialbahn 12 die Ränder der spaltförmigen Öffnungen durch Ränder abnehmbarer oder abklappbarer Gehäuseelemente gebildet werden. Ist, wie in Fig. 2b gezeigt, das Gehäuse 22 an das Gehäuse 24 der Zuführeinheit 18 angekoppelt, kann die Zuführeinheit 18 der Wickeleinheit 16 beim Abwickeln zum Aufbringen eines festen, flüssigen oder gasförmigen Stoffes 20b nachgeschaltet werden. Dies kann insbesondere dann vorteilhaft sein, wenn der feste, flüssige oder gasförmige Stoff 20b erst unmittelbar vor dem Anlegen auf die Materialbahn 12 aufgebracht werden soll, bspw. weil der feste, flüssige oder gasförmige Stoff 20b flüchtig ist, die Materialbahn 12 aushärtet (Wasser auf Gipsbinden), etc.

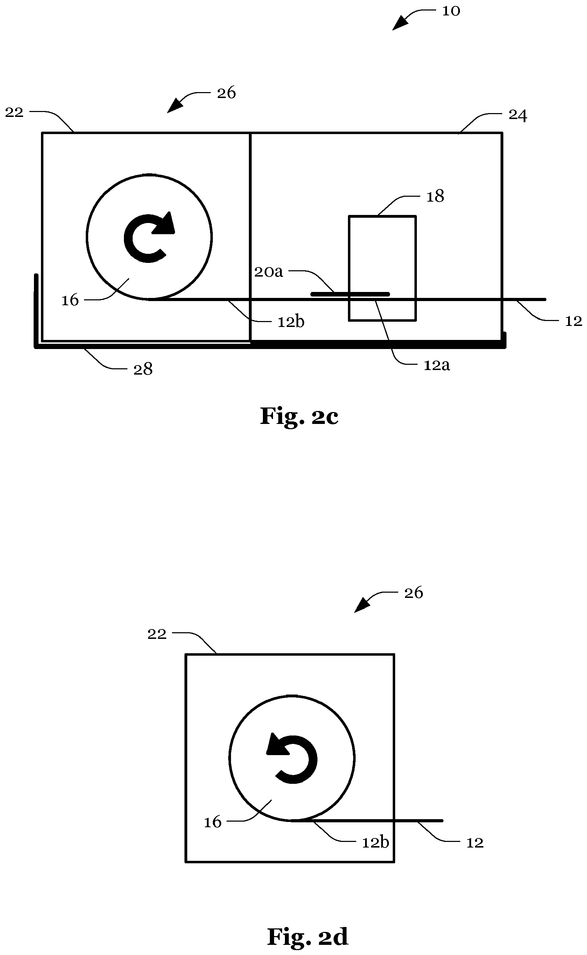

Ferner kann, wie in Fig. 2c gezeigt, die Zuführeinheit 18 der Wickeleinheit 16 beim Aufwickeln zum Aufbringen eines festen, flüssigen oder gasförmigen Stoffes 20b vorgeschaltet werden, wenn das Gehäuse 22 an das Gehäuse 24 der Zuführeinheit 18 angekoppelt ist. Dies kann insbesondere dann vorteilhaft sein, wenn der feste, flüssige oder gasförmige Stoff 20a eine bestimmte Zeit vor dem Anlegen auf die Materialbahn 12 aufgebracht werden soll, bspw. weil der feste, flüssige oder gasförmige Stoff 20a einer bestimmten Vorlaufs- oder Einwirkzeit (vor dem Anlegen) bedarf, besonders gleichmäßig über die Materialbahn 12 verteilt werden soll, das Gehäuse 24 der Zuführeinheit 18 oder die Zuführeinheit 18 selbst beim Anlegen der Materialbahn 12 stören würde, etc.

Ferner versteht es sich, dass dieselbe oder unterschiedliche Zuführeinheiten 18 der Wickeleinheit 16 beim Aufwickeln und/oder Abwickeln zum Aufbringen eines festen, flüssigen oder gasförmigen Stoffes 20a, 20b vorgeschaltet/nachgeschaltet werden können, so dass die in Fig. 2a-2d gezeigte Abwandlung neben den Merkmalen/Verwendungsmöglichkeiten der in Fig. 1a und 1b gezeigten Vorrichtung 10 (unter anderem) noch die Möglichkeit birgt, auf mehrere übereinander gewickelte Materialbahnen 12 durch Tauschen der Zuführeinheiten 18 unterschiedliche feste, flüssige oder gasförmige Stoffe 20a, 20b aufzubringen. Wenn die Zuführeinheiten 18 keines "Einfädelns" der Materialbahn 12 bedürfen bzw. die Materialbahn 12 ohne Zertrennen aus der Zuführeinheit 18 ausgefädelt werden kann, können die Zuführeinheiten 18 zudem auch während des Aufwickelns und/oder Abwickelns einer Materialbahn 12 getauscht bzw. an- oder abgekoppelt werden.

Zudem versteht es sich, dass durch den modularen Aufbau und die Möglichkeit der Verwendung unterschiedlicher Zuführeinheiten 18 neben der preiswerteren Ersetzbarkeit der Module auch insofern Vorteile entstehen, als je nach Aufbringungsbedarf, auf bestimmte Einsatzszenarien angepasste (spezialisierte) Zuführeinheiten 18 verwendet werden können, bspw. Zuführeinheiten 18, die ein besonders schnelles Aufwickeln unterstützen, Zuführeinheiten 18, die feste, flüssige oder gasförmige Stoffe 20a, 20b nur auf bestimmte Bereiche der Materialbahn 12 aufbringen, Zuführeinheiten 18, die im Wechsel feste, flüssige oder gasförmige Stoffe 20a, 20b aufbringen können, Zuführeinheiten 18, die besonders robust gegen bestimmte feste, flüssige oder gasförmige Stoffe 20a, 20b sind (bspw. Zuführeinheiten 18 aus Edelstahl), Zuführeinheiten 18, die besonders einfach zu reinigen/sterilisieren sind, etc. Ferner kann eine einzelne Zuführeinheit 18 dazu benutzt werden, mehrere Wickeleinheiten 16 (nacheinander) mit Materialbahnen 12 zu bestücken.

Des Weiteren kann das Gehäuse 22 mit dem in dem Gehäuse 22 drehbar gelagerten Element der Wickeleinheit 16 im abgekoppelten Zustand auch zum Auf- und Abwickeln der Materialbahn 12 ohne Aufbringen eines festen, flüssigen oder gasförmigen Stoffes 20a, 20b, verwendet werden, wie in Fig. 2a und 2d gezeigt. Insofern kann das Gehäuse 22 mit dem in dem Gehäuse 22 drehbar gelagerten Element der Wickeleinheit 16 als eigenständige Vorrichtung 26 verwendet werden. Dabei versteht es sich, dass die Vorrichtung 26 Elemente zum Ankoppeln an das Gehäuse 24 der Zuführeinheit 18 aufweisen kann, aber nicht muss. Bspw. kann die Zuführeinheit 18, wie in Fig. 2b und 2c gezeigt, eine Halterung 28 aufweisen, die an die äußere Form der Vorrichtung 26 angepasst ist, so dass die Halterung 28 in angekoppeltem Zustand das Gehäuse 22 (teilweise) umgreift.

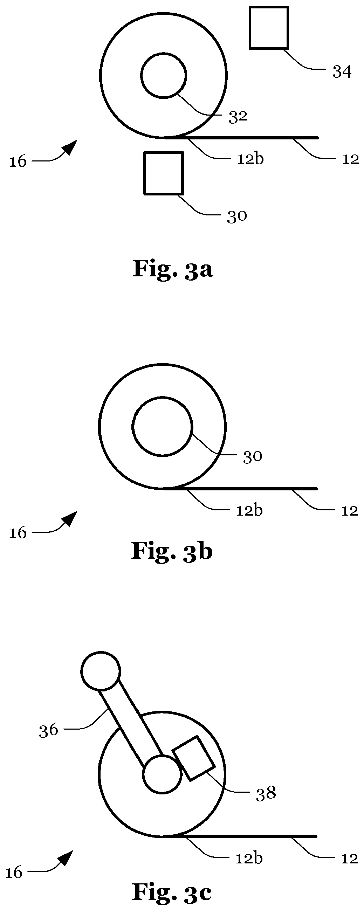

Fig. 3a zeigt eine schematische Darstellung einer beispielhaften Ausgestaltung der Wickeleinheit 16. Die in Fig. 3a gezeigte Wickeleinheit 16 umfasst eine Energiespeichereinheit 30, der zum Drehen des drehbar gelagerten Elements Energie entnommen werden kann. Bspw. kann die Energiespeichereinheit 30 eine oder mehrere Primär- oder Sekundärzellen umfassen, durch die ein mit dem drehbar gelagerten Element bzw. einer Welle des drehbar gelagerten Elements verbundener elektrischer Motor 32 mit Energie gespeist werden kann. Dabei versteht es sich, dass die Wickeleinheit 16 anstatt einer (oder zusätzlich zur) Energiespeichereinheit 30 auch elektrische Anschlüsse zum Anschluss an eine externe Energieversorgung aufweisen kann. Bspw. kann die Wickeleinheit 16 oder die Energiespeichereinheit 30 zum Anschluss an ein Stromnetz eingerichtet sein.

Der elektrische Motor 32 kann mit einer Steuereinheit 34 verbunden sein, die eine Einstellung der Aufwickelgeschwindigkeit ermöglicht, bspw. über ein oder mehrere am Gehäuse 14, 22 angebrachte Eingabeelemente, wie z. B. Schalter oder Taster. Um eine möglichst konstante Einziehgeschwindigkeit zu erreichen, kann die Steuereinheit 34 ferner dazu eingerichtet sein, die Drehgeschwindigkeit des drehbar gelagerten Elements im Laufe des Aufwickelvorgangs schrittweise oder kontinuierlich zu reduzieren oder zu steigern. Bspw. kann die Dicke der Materialbahn 12 über ein oder mehrere am Gehäuse 14, 22 angebrachte Eingabeelemente, wie z. B. Schalter, durch den Benutzer eingegeben werden, oder die Dicke des Wickels mittels eines Sensors bestimmt werden. Insbesondere kann die Steuereinheit 34 mit verschiedenen Programmen zum Aufwickeln unterschiedlicher Typen an Materialbahnen 12 versehen sein, wobei der Benutzer über ein oder mehrere am Gehäuse 14, 22 angebrachte Eingabeelemente, wie z. B. Schalter oder Taster, zwischen den Programmen wählen kann.

Fig. 3b zeigt eine schematische Darstellung einer weiteren beispielhaften Ausgestaltung der Wickeleinheit 16, welche sich von der in Fig. 3a gezeigten Ausgestaltung dadurch unterscheidet, dass die Energiespeichereinheit 30 anstatt Primär- oder Sekundärzellen eine Konstantkraftfeder 90 aufweist, der durch Drehen des drehbar gelagerten Elements in Abwickelrichtung Energie zugeführt und durch Drehen des drehbar gelagerten Elements in Aufwickelrichtung Energie entnommen werden kann. Dadurch ist ein selbsttätiges Aufwickeln der Materialbahn 12 möglich, indem nach dem Abwickeln eine Materialbahn 12 an dem drehbar gelagerten Element befestigt und mittels durch die Konstantkraftfeder 90 bereitgestellter Energie eingezogen und aufgewickelt wird.

Fig. 3c zeigt eine schematische Darstellung einer weiteren beispielhaften Ausgestaltung der Wickeleinheit 16, welche sich von den in Fig. 3a und 3b gezeigten Ausgestaltungen dadurch unterscheidet, dass anstatt der Energiespeichereinheit 30 eine Kurbel 36, 102 vorgesehen ist, die ein manuell betätigtes Drehen des drehbar gelagerten Elements zum Aufwickeln und Einziehen der Materialbahn 12 ermöglicht. Dabei kann vorgesehen sein, dass die Kurbel 36, 102 abnehmbar, samt der Welle 74 des drehbar gelagerten Elementes aus dem Gehäuse 14, 24 herausausziehbar oder von dem drehbar gelagerten Element entkoppel- und an das Gehäuse 14, 24 anklappbar ist, so dass die Kurbel 36, 102 beim Abwickeln der Materialbahn 12 nicht mitdreht. Um beim Abwickeln der Materialbahn eine (annähernd) konstante Zugkraft zu erreichen, kann die Wickeleinheit 16 mit einer einstellbaren Bremse 38 versehen sein.

Ferner können die in Fig. 3a bis 3c gezeigten Bauelemente der Wickeleinheiten 16 auch in einer Wickeleinheit 16 kombiniert sein. Bspw. kann vorgesehen sein, dass in der in Fig. 3b gezeigten Ausführungsform die Konstantkraftfeder 90 mittels einer Kurbel 36, 102 (nach-) gespannt werden kann, bspw. bei einem initialen Aufwickeln oder wenn vom Aufwickeln kürzerer Materialbahnen 12 auf das Aufwickeln längerer Materialbahnen 12 umgestellt wird. Zudem kann in der in Fig. 3b gezeigten Ausführungsform ein elektrischer Motor 32 zur Unterstützung der Konstantkraftfeder 90 vorgesehen sein, oder in der in Fig. 3c gezeigten Ausführungsform eine einstellbare Bremse 38 zur Erzielung einer (annähernd) konstanten Zugkraft beim Abwickeln.

Des Weiteren können die in Fig. 1a bis 3c gezeigten Wickeleinheiten 16 eingerichtet sein, eine die Drehung des in dem Gehäuse 14, 22 drehbar gelagerten Elements verhindernde Sperrung aufzuheben, wenn auf den durch das Gehäuse 14, 22 geführten und an dem drehbaren Element befestigten Materialbahnabschnitt 12b eine Zugspannung ausgeübt wird, die oberhalb einer bestimmten Zugspannungsschwelle liegt. Bspw. kann in den in Fig. 3a und 3c gezeigten Ausführungsformen die Zugspannung mittels eines Sensors gemessen werden und das drehbare Element von dem elektrischen Motor 32 entkoppelt bzw. die Bremse gelöst werden, wenn die Zugspannung oberhalb der bestimmten Zugspannungsschwelle liegt.

Alternativ kann die in den in Fig. 3a-3c gezeigten Ausführungsformen die Drehung des in dem Gehäuse 14, 22 drehbar gelagerten Elements verhindernde Sperrung durch eine (rein) mechanische Anordnung mit einem mechanischen Sperrelement erzielt werden, wobei ein Eingriff des Sperrelements gelöst wird, wenn die Zugspannung oberhalb der bestimmten Zugspannungsschwelle liegt. Bspw. kann der Materialbahnabschnitt 12b bei Zugspannung eine gleitend gelagerte Rolle mit einer Kraft beaufschlagen, so dass die Rolle gegen ein elastisches Element verschoben wird, wobei das Verschieben der Rolle den Eingriff des Sperrelements in das drehbar gelagerte Element oder eine Welle des drehbar gelagerten Elements löst.

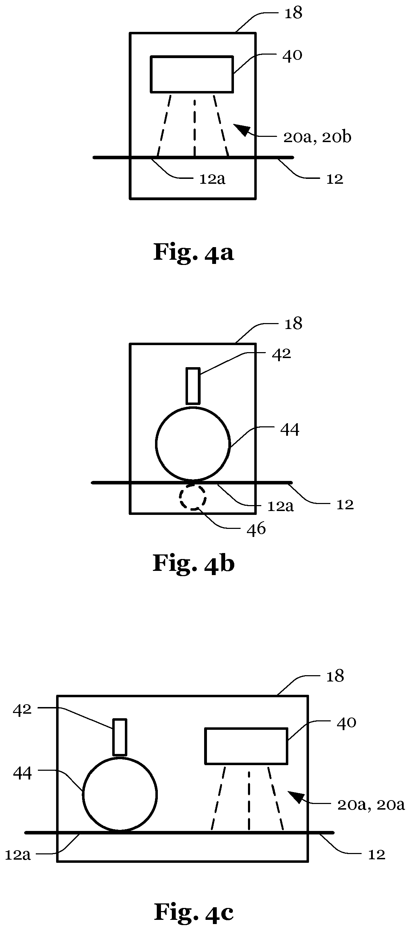

Fig. 4a zeigt eine schematische Darstellung einer beispielhaften Ausgestaltung der Zuführeinheit 18 der in Fig. 1a, 1b, 2b, und 2c gezeigten Vorrichtungen 10. Die Zuführeinheit 18 umfasst eine Zerstäubungs-/Nebeldüse 40, die eingerichtet ist, einen festen oder flüssigen Stoff 20a, 20b zu zerstäuben, wobei der dabei entstehende Stoffstrom auf den die Zuführeinheit 18 passierenden Materialbahnabschnitt 12a gelenkt wird. Anstatt oder zusätzlich zur Zerstäubungs-/Nebeldüse 40 kann die Zuführeinheit 18 auch einen Auslass zum Einbringen eines gasförmigen Stoffes 20a, 20b in die Zuführeinheit 18 umfassen, wobei der die Zuführeinheit 18 passierende Materialbahnabschnitt 12a durch die sich dabei ausbildende Atmosphäre geführt wird.

Fig. 4b zeigt eine schematische Darstellung einer weiteren beispielhaften Ausgestaltung der Zuführeinheit 18 der in Fig. 1a, 1b, 2b, und 2c gezeigten Vorrichtungen 10. Die Zuführeinheit 18 umfasst eine Flachdüse 42, die einen festen oder flüssigen Stoff 20a, 20b auf eine Rolle 44 aufträgt, welche den festen oder flüssigen Stoff 20a, 20b auf den die Zuführeinheit 18 passierenden Materialbahnabschnitt 12a überträgt. Wie in Fig. 4b gezeigt, kann die Materialbahn 12 dabei durch einen Spalt zwischen der Rolle 44 und einem Gegendruckzylinder 46 geführt werden. Gegendruckzylinder 46 und Rolle 44 können zudem bspw. mittels eines Getriebes synchronisiert sein, um ein Gleiten der Materialbahn 12 über die Oberfläche der Rolle 44 beim Auftragen von Stoffen eines bestimmten Viskositätsbereichs zu verhindern. Zusätzlich zur Düse 42 kann die Zuführeinheit 18 auch einen Auslass zum Einbringen eines gasförmigen Stoffes 20a, 20b in die Zuführeinheit 18 umfassen, wobei der die Zuführeinheit 18 passierende Materialbahnabschnitt 12a durch die sich dabei ausbildende Atmosphäre geführt wird.

Wie in Fig. 4c gezeigt, können die in Fig. 4a und 4b gezeigten Elemente auch in einer Zuführeinheit 18 kombiniert werden. Ferner versteht es sich, dass in einer der in den Fig 4a-4c gezeigten Zuführeinheiten 18 anstatt einer Zerstäubungs-/Nebeldüse 40, einer Flachdüse 42 oder einer Rolle 44 auch mehrere Zerstäubungs-/Nebeldüsen 40, Flachdüsen 42 oder Rollen 44 vorgesehen sein können. Ferner können, wie weiter oben angemerkt, die Düsen 40, 42 eingerichtet sein, den Stoffstrom nur auf Teilbereiche des die Zuführeinheit 18 passierenden Materialbahnabschnitts 12a zu lenken, bspw. indem die Düsen 40, 42 geschwenkt oder (teilweise) verschlossen/deaktiviert werden. Zudem kann eine Stoffzufuhr zur Zuführeinheit 18, bzw. zu einerr oder mehreren Zerstäubungs-/Nebeldüsen 40 und/oder zu einer oder mehreren Flachdüsen 42 steuerbar und insbesondere mit der Geschwindigkeit der Materialbahn 12 synchronisierbar sein.

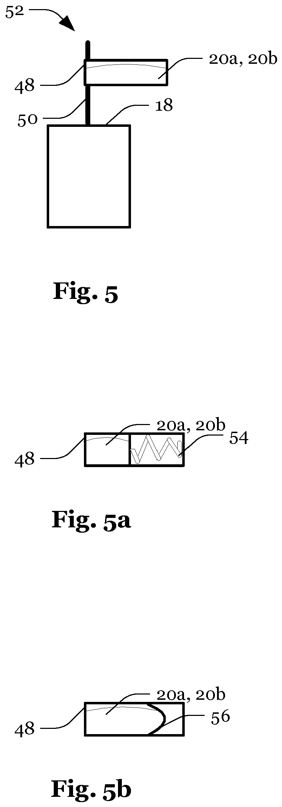

Fig. 5 zeigt eine schematische Darstellung einer beispielhaften Ausgestaltung eines mit der Zuführeinheit 18 verbundenen Vorratsbehälters 48, der in dem Gehäuse 14, 24 angeordnet sein kann. Zwischen dem Vorratsbehälter 48 und der Zuführeinheit 18 kann eine oder mehrere Leitungen 50 vorgesehen sein, mittels derer die Zuführeinheit 18 gespeist wird. Zum Wiederauffüllen des Vorratsbehälter 48 kann das Gehäuse 14, 24 bspw. eine Öffnung bzw. einen Anschluss 52 aufweisen, über den der Vorratsbehälter 48 befüllt werden kann. Alternativ kann der Vorratsbehälter 48 aus dem Gehäuse 14, 24 entnehmbar oder von dem Gehäuse 14, 24 abkoppelbar sein.

Um den Füllstand des Vorratsbehälters 48 bestimmen zu können, kann das Gehäuse 14, 24 bzw. der Vorratsbehälter 48 einen durchsichtigen Abschnitt aufweisen, durch den ein Einblick in den Vorratsbehälter 48 möglich ist. Ferner kann der Vorratsbehälter 48 mit einer an der Außenseite des Gehäuses 14, 24 angeordneten Druckanzeige verbunden sein. Die Druckanzeige kann dabei sowohl verwendet werden, wenn der Vorratsbehälter 48 mit einem gasförmigen Stoff 20a, 20b druckbefüllt wird/ist, als auch, wenn das Volumen des Vorratsbehälters 48 beim Befüllen mit einem flüssigen Stoff 20a, 20b mittels Verformung eines elastischen Elements 54 vergrößert wird und der flüssige Stoff 20a, 20b dementsprechend mit einer durch die Elastizität des elastischen Elements 54 hervorgerufenen Kraft beaufschlagt wird. Das elastische Element 54 kann bspw., wie in Fig. 5a gezeigt, eine Feder sein, die an einer verschieblich gelagerten Wand des Vorratsbehälters 48 angeordnet ist, oder, wie in Fig. 5b gezeigt, eine elastische Wand 56 des Vorratsbehälters 48.

Der Vorratsbehälter 48 kann ferner außerhalb des Gehäuses 14, 24 angeordnet sein. Bspw. kann die Leitung 50 in einen Anschluss (am Gehäuse 14, 24) münden, über den der Vorratsbehälter 48 mit der Zuführeinheit 18 verbunden werden kann. Ferner kann die Zuführeinheit 18 mit mehreren Vorratsbehältern 48 verbunden sein, wobei die Vorratsbehälter 48 innerhalb oder außerhalb des Gehäuses 14, 24 angeordnet sein können. Bspw. kann die Zuführeinheit 18 beim Einziehen der Materialbahn 12 einen ersten festen, flüssigen oder gasförmigen Stoff 20a auf die Materialbahn 12 aufbringen, wobei der erste feste, flüssige oder gasförmige Stoff 20a aus einem Vorratsbehälter 48 außerhalb des Gehäuses 14, 24 bereitgestellt wird. Des Weiteren kann der Vorratsbehälter 48 auch drucklos sein, wobei sein Inhalt angesaugt und mittels einer Pumpe zur Zuführeinheit 18 gefördert wird.

Zudem kann die Zuführeinheit 18 beim Einziehen der Materialbahn 12 einen zweiten festen, flüssigen oder gasförmigen Stoff 20a auf die Materialbahn 12 aufbringen, wobei der zweite feste, flüssige oder gasförmige Stoff 20a aus einem Vorratsbehälter 48 innerhalb des Gehäuses 14, 24 bereitgestellt wird. Bspw. kann das Aufbringen des zweiten festen, flüssigen oder gasförmigen Stoff 20a einen geringeren Volumenstrom bedingen, als das Aufbringen des ersten festen, flüssigen oder gasförmigen Stoffes 20a, der zweite feste, flüssige oder gasförmige Stoff 20a länger haltbar sein, als der erste feste, flüssige oder gasförmige Stoff 20a, der der zweite feste, flüssige oder gasförmige Stoff 20a robuster gegen Temperaturschwankungen sein, als der erste feste, flüssige oder gasförmige Stoff 20a, etc.

Ferner kann die Zuführeinheit 18 den zweiten festen, flüssigen oder gasförmigen Stoff 20b, wie in Fig. 1b gezeigt, beim Abwickeln der Materialbahn 12 auf die Materialbahn 12 aufbringen, wobei der zweite feste, flüssige oder gasförmige Stoff 20b aus einem Vorratsbehälter 48 innerhalb der Vorrichtung 10 bereitgestellt wird. Bspw. kann das Aufbringen des zweiten festen, flüssigen oder gasförmigen Stoffes 20b unmittelbar vor dem Anlegen der Materialbahn 12 erfolgen.

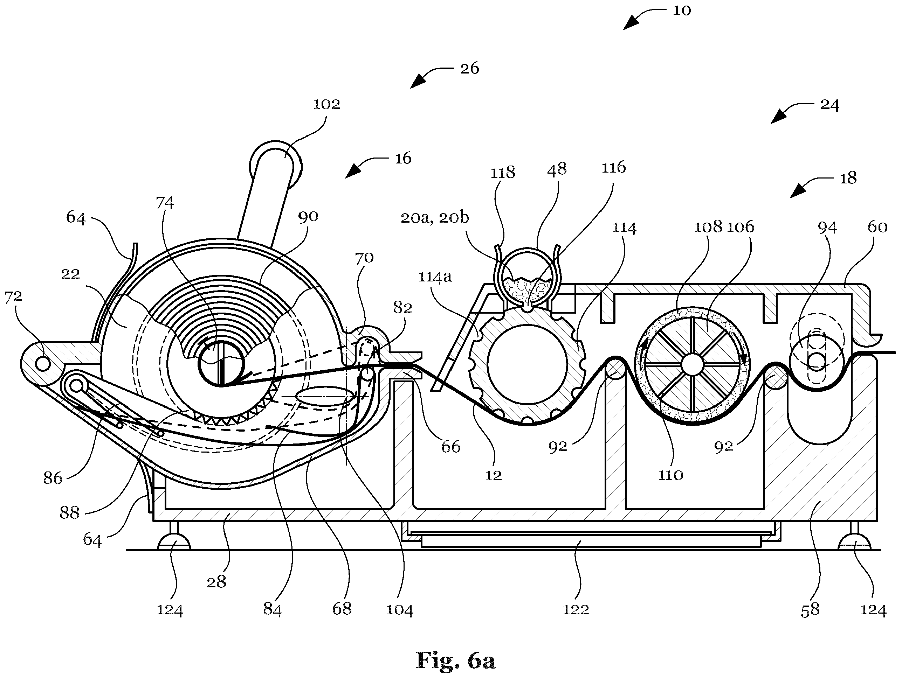

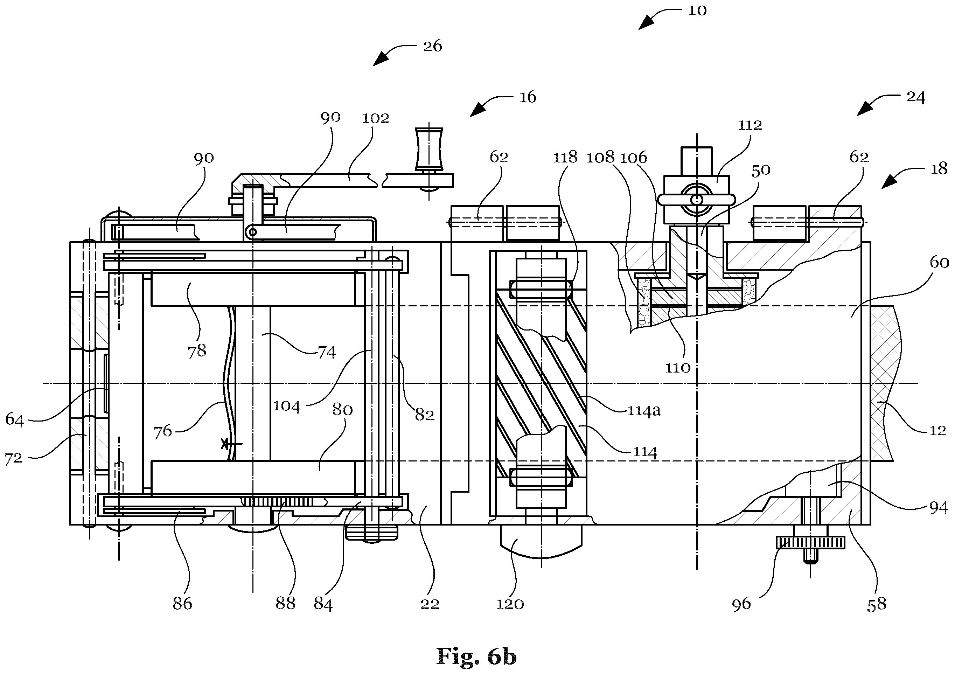

Fig. 6a und 6b zeigen eine beispielhafte Ausgestaltung der in Fig. 2a-2d gezeigten modularen Bauweise der Vorrichtung 10 bzw. eine beispielhafte Ausgestaltung der Vorrichtung 26. Wie in Fig. 6a gezeigt, kann das Gehäuse 24 der Zuführeinheit 18 ein Unterteil 58 und ein Oberteil 60 aufweisen, welche, wie in Fig. 6b gezeigt, mittels eines oder mehrerer Scharniere 62 miteinander verbunden sein können. Das Unterteil 58 kann ferner eine Halterung 28 zum Ankoppeln des Gehäuses 22 der Wickeleinheit 16 aufweisen. Wie in Fig. 6a gezeigt, kann das Gehäuse 22 der Wickeleinheit 16 zum Ankoppeln so in die Halterung 28 eingebracht werden, dass das Gehäuse 22 der Wickeleinheit 16 von einer Federklammer 64 zentriert und gehalten wird. Um eine Drehung des Gehäuses 22 um die Wickelachse zu unterbinden, kann ferner eine, einen Rand der spaltförmigen Öffnung des Gehäuses 22 bildende Lippe 66 an einer Stütze des Unterteils 58 anliegen.

Wie in Fig. 6a gezeigt, kann das Gehäuse 22 der Wickeleinheit 16 ebenfalls ein Unterteil 68 und ein Oberteil 70 aufweisen, welche mittels eines oder mehrerer Scharniere 72 miteinander verbunden sein können. Zum Aufwickeln der Materialbahn 12 kann ferner ein Ende der Materialbahn 12 bei geöffnetem Gehäuse 22 in eine, in dem Gehäuse 22 drehbar gelagerte Welle 74 der Wickeleinheit 16 eingefädelt bzw. mit einer Klammer 76 (siehe Fig. 6b) zwischen den Leitseiten 78, 80 der Welle 74 verankert und über die Lippe 66 geführt werden.

Die lockere Materialbahn 12 kann dann über einen Querstab 82 geführt werden, der mit Zahnhebeln 84 verbunden ist, die mittels Schenkelfedern 86 nach oben gehaltenen werden und in dieser Position in Zahnscheiben 88 eingreifen, wobei durch den Eingriff ein Entspannen einer mit der Welle 74 verbundenen Konstantkraftfeder 90 unterbunden wird. Wie in Fig. 6a gezeigt, können die Zahnhebel 84 im Unterteil 68 des Gehäuses 22 drehbar gelagert sein. Ferner kann die Konstantkraftfeder 90 im Unterteil 68 des Gehäuses 22 an einer Gehäusestirnseite angeordnet und mit einem Ende an der Welle 74 und dem anderen Ende am Unterteil 68 des Gehäuses 22 befestigt sein.

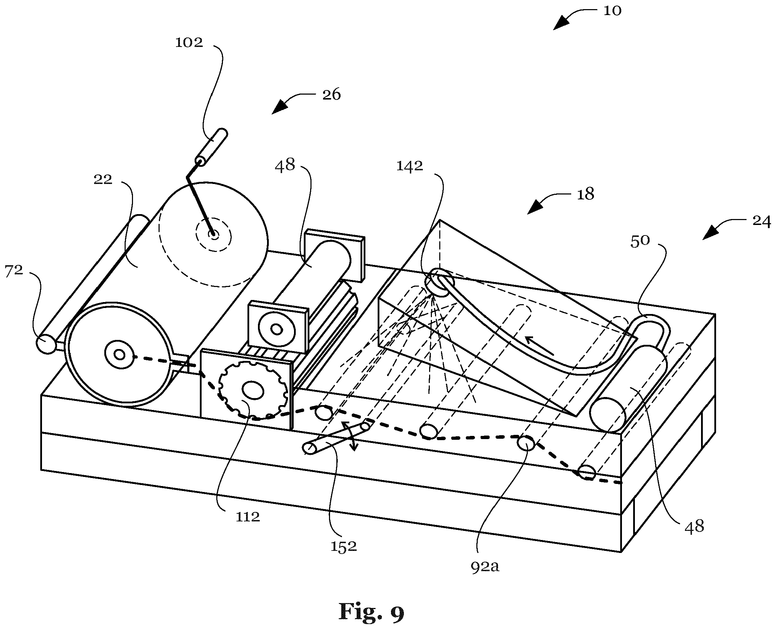

Bei geöffnetem Gehäuse 24 kann die Materialbahn 12 dann über (das Unterteil 58 untergliedernde) Stege 92 geführt werden, die bspw., wie in Fig. 9 gezeigt, auch als Querstäbe 92a ausgeführt sein können. Danach kann das Gehäuse 24 geschlossen werden, wodurch die im Oberteil 60 angeordnete Zuführeinheit 18 in ihre Arbeitsposition über der Materialbahn 12 bewegt wird. Durch das Schließen des Gehäuses 24 kann ferner eine ebenfalls im Oberteil 60 angeordnete einstellbare Spannrolle 94 zum Spannen der Materialbahn 12 in ihre Arbeitsposition über der Materialbahn 12 bewegt werden. Wie in Fig. 6b gezeigt, kann die Bremskraft der Spannrolle 94 mittels zweier auf den Außenseiten des Gehäuses 24 gleitenden Spannmuttern 96 einstellbar sein.

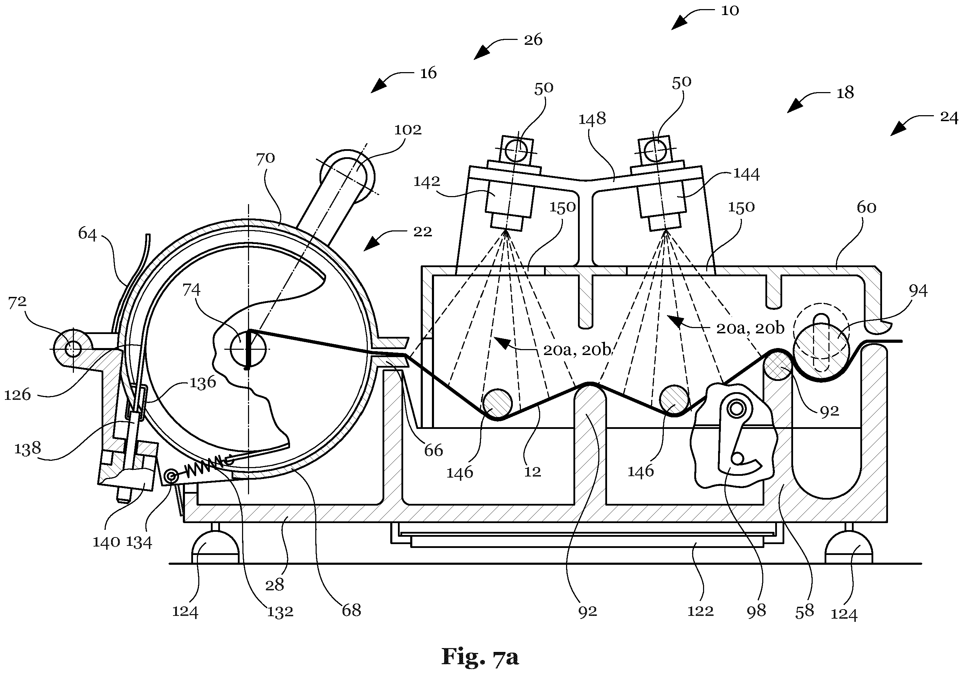

Nach dem Schließen des Gehäuses 24 (und einem Sichern des Gehäuses 24 mit einem Schnappverschluß oder einem Schließhaken 98, wie in Fig. 7a gezeigt), kann durch Anspannen der Materialbahn 12 der Querstab 82 nach unten gedrückt werden, so dass der Eingriff der Zahnhebel 84 in die Zahnscheiben 88 der Welle 74 gelöst wird. Dadurch kann sich die Konstantkraftfeder 90 entspannen, wobei durch die dabei zur Verfügung gestellte Energie ein Aufwickeln der Materialbahn 12 um die Welle 74 bewirkt werden kann. Sobald das andere Ende der Materialbahn 12 die Spannrolle 94 passiert hat, wird die Beaufschlagung des Querstabs 82 aufgehoben und die Schenkelfedern 86 bewirken eine Verzahnung der Zahnhebel 84 mit den Zahnscheiben 88, so dass die Welle 74 wieder gesperrt wird.

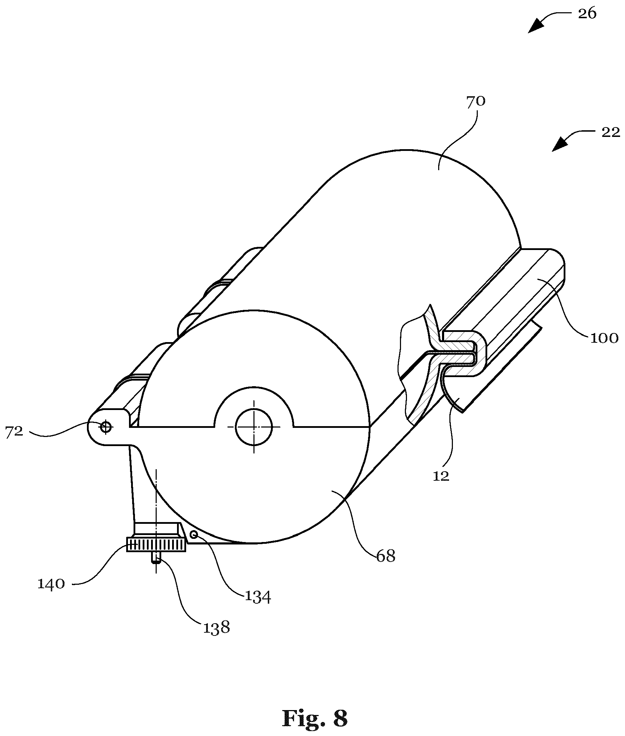

Nach dem Aufwickeln kann die Wickeleinheit 16 von der Zuführeinheit 18 abgekoppelt werden und optional zur (keimfreien) Lagerung verschlossen werden, bspw. mit einem (Aufschnapp-) Deckel 100 (wie in Fig. 8 gezeigt). Die Wickeleinheit 16 kann ferner nach Abnehmen des (Aufschnapp-) Deckels 100 und Herausziehen der Materialbahn 12 gegen den Widerstand der Konstantkraftfeder 90 zum Anlegen der Materialbahn 12 genutzt werden. Dies gewährleistet eine konstante Spannung der Materialbahn 12 beim Anlegen und ermöglicht zugleich ein Spannen der Konstantkraftfeder 90. Wenn die Materialbahn 12 aus dem Gehäuse 22 vollständig herausgezogen ist, wird der Querstab 82 freigegeben und die Schenkelfedern 84 bewirken ein Einrasten der Verzahnungen in die Zahnscheiben 88 der Welle 74, so dass die Konstantkraftfeder 90 für das Einziehen der nächsten Materialbahn 12 gespannt bleibt.

Zum Spannen der Konstantkraftfeder 90 kann ferner, wie in Fig. 6a gezeigt, eine mit der Welle 74 verbundene Kurbel 102 vorgesehen sein. Ferner kann eine Exzenterachse 104 vorgesehen sein, die bei Drehung eines Griffes die Zahnhebel 84 gegen die Kraft der Schenkelfedern 86 zurückdrückt und dadurch die Verzahnung der Zahnhebel 84 mit den Zahnscheiben 88 aufhebt. Nach Spannen der Konstantkraftfeder 90 kann die Exzenterachse 104 wieder zurückgedreht werden, wodurch die Zahnhebel 84 wieder zur Verzahnung mit den Zahnscheiben 88 freigegeben werden.

Zum Desinfizieren der eingezogenen Materialbahn 12 kann in der Zuführeinheit 18 ein Desinfektionsmittel-Geber 106 mit einer Rolle 108 vorgesehen sein. Der Kern des Desinfektionsmittel-Gebers 106 kann bspw., wie in Fig. 6a gezeigt, radiale Kapillarkanäle 110 aufweisen, die, wie in Fig. 6b gezeigt, über eine in einen abschließbaren Hahn 112 mündende Leitung 50 mit einem Desinfektionsmittel aus einem Vorratsbehälter 48 versorgt werden können. Werden die Kapillarkanäle 110 mit dem Desinfektionsmittel versorgt, kann dieses von der den Kern ummantelnden porösen, halbelastischen Rolle 108 aus den Kapillarkanälen 110 aufgenommen werden.

Die poröse, halbelastische Rolle 108, die bspw. aus einem Schaummaterial gefertigt sein kann, kann auf dem Kern des Desinfektionsmittel-Gebers 106 gleitend drehbar gelagert sein, so dass sie durch das Einziehen der Materialbahn 12 um den Kern des Desinfektionsmittel-Gebers 106 gedreht wird, wobei ein Teil der von der Rolle 108 aufgenommenen Flüssigkeit beim Kontakt mit der Materialbahn 12 auf diese übertragen wird. Beim Passieren des folgenden Stegs 92 kann zudem überflüssiges Desinfektionsmittel aus der Materialbahn 12 herausgedrückt bzw. von der Materialbahn 12 abgestreift werden. Dabei versteht es sich, dass der Desinfektionsmittel-Geber 106 auch zum Aufbringen anderer flüssiger Stoffe 20a, 20b eingesetzt werden kann.

Die gespannte Materialbahn 12 kann zudem eine dem Desinfektionsmittel-Geber 106 beim Aufwickeln nachgeschaltete Aufnahme- und Verteilerrolle 114 antreiben. Die Aufnahme- und Verteilerrolle 114 kann bspw. (spiralartige) Einkerbungen 114a aufweisen, in die beim Passieren von Spenderbohrungen 116 eines darüberliegenden Vorratsbehälters 48, ein in Pulverform bevorrateter fester Stoff 20a, 20b, wie bspw. ein (Wund-) Heilmittel, wie z. B. EGCG, fällt. Die Spenderbohrungen 116 können ferner der Länge nach und nur auf einer Seite des rohrartigen Vorratsbehälters 48 vorgesehen sein.

Wie in Fig. 6a gezeigt, kann der Vorratsbehälters 48 am Gehäuse 24 der Zuführeinheit 18 angekoppelt sein. Zum Ankoppeln kann der Vorratsbehälter 48 bspw., wie in Fig. 6a gezeigt, zwischen zwei Aufnahme-Klemmfedern 118 geschoben werden. Die Aufnahme-Klemmfedern 118 fixieren den Vorratsbehälter 48 und drücken diesen gegen die Aufnahme- und Verteilerrolle 114. Die Spenderbohrungen 116 des Vorratsbehälters 48 können vor Gebrauch mit einer Schutzfolie überzogen sein, die vor dem Einschieben des Vorratsbehälters 48 zu entfernen ist. Beim Einsetzen des Vorratsbehälters 48 und in Verwendungspausen kann der Vorratsbehälters 48 so gedreht werden, dass die Spenderbohrungen 116 nicht vertikal nach unten ausgerichtet sind, um ein Herausfallen des in Pulverform bevorrateten Heilmittels zu verhindern. Um die Abgabe des in Pulverform bevorrateten Heilmittels zu ermöglichen, kann der Vorratsbehälters 48 bspw. mittels eines Spendergriffs 120 so gedreht werden, dass die Spenderbohrungen 116 vertikal nach unten ausgerichtet sind.

Das in den Einkerbungen 114a befindliche Heilmittel kann dann an die die Aufnahme- und Verteilerrolle 114 passierende Materialbahn 12 abgegeben werden. Die Aufnahme- und Verteilerrolle 114 kann ferner aus einem Flüssigkeit abweisenden Material gefertigt und/oder mit Polytetrafluorethylen, PTFE, beschichtet sein. Zur Aufnahme von festen oder flüssigen Stoffen 20a, 20b kann unter der Zuführeinheit 18 zudem ein Auffangtablett 122 vorgesehen sein. Des Weiteren kann das Gehäuse 24 an der Unterseite (bzw. an den Gehäuseecken) mit Saugnäpfen 124 versehen sein.

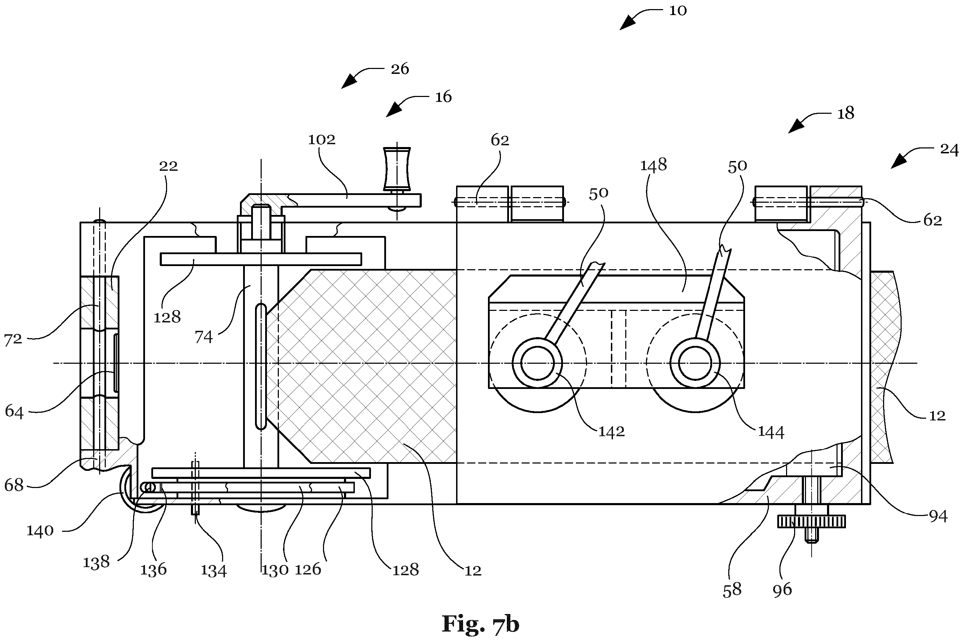

Fig. 7a und 7b zeigen eine im Vergleich zu der in Fig. 6a und 6b gezeigten Ausgestaltung modifizierte Ausgestaltung, welche sich von der in Fig. 6a und 6b gezeigten Ausgestaltung (unter anderem) dadurch unterscheidet, dass die Konstantkraftfeder 90 durch ein Bremsband 126 ersetzt ist und die Zahnhebel 84 und Zahnscheiben 88 entfallen. Wie in Fig. 7b gezeigt, kann dazu stirnseitig an einem der Aufwickel-Leitflansche 128 eine Bremstrommel 130 angeordnet sein, welche das Bremsband 126 aufnimmt. Das Bremsband 126 kann ferner an einem Ende mit einer Spannfeder 132 versehen sein, die an einem Federstift 134 verankert ist. Am anderen Ende kann das Bremsband 126 mittels eines Ankerhakens 136 an einer Gewindestange 138 mit einer außenliegenden Spannmutter 140 befestigt sein, die, wie in Fig. 8 gezeigt, ein Einstellen der Bremskraft bei geschlossenem Gehäuse 22 ermöglicht.

Das Aufwickeln der Materialbahn 12 nach dem Einfädeln kann mittels der abnehmbaren Kurbel 102 unter entlasteter Spannung der Bremsfeder 132 erfolgen. Beim Abwickeln der Materialbahn 12 kann die Spannmutter 140 zur individuellen Einstellung der Bremswirkung und somit der gewünschten Materialbahnspannung, sowie zum Verhindern eines ungewollten Abwickelns der Materialbahn 12 verwendet werden. Fig. 7a und 7b zeigen zudem eine modifizierte Ausgestaltung der Zuführeinheit 18 mit zwei Nebeldüsen 142, 144 zum Besprühen der Materialbahn 12 mit flüssigen Stoffen 20a, 20b, wie bspw. einem Desinfektionsmittel und einem Heilmittel in flüssiger Form. Zudem kann die Vorrichtung 10, wie in Fig. 7a gezeigt, anstatt des Desinfektionsmittel-Gebers 106 und der Aufnahme- und Verteilerrolle 112 Führungsstäbe oder - flächen 146 umfassen, die zur Spannung und Führung der Materialbahn 12 dienen.

Wie in Fig. 7a gezeigt, können die Nebeldüsen 142, 144 auf einer Halterung 148 auf dem Oberteil 60 des Gehäuses 24 abnehmbar montiert sein. Dabei können (runde oder eckige) Öffnungen 150 im Oberteil 60 zur Steuerung und Begrenzung des Stoffstroms dienen. Wie in Fig. 7b gezeigt, können die Nebeldüsen 142, 144 über Leitungen 50 von unter Druck stehenden, außerhalb des Gehäuses 24 angeordneten Vorratsbehältern 48 gespeist werden und bspw. über Hähne 112 nach Bedarf ein- und abschaltbar sein.

Des Weiteren können, wie in Fig. 9 gezeigt, der oder die Vorratsbehälter 48 in dem Gehäuse 24 angeordnet sein, wobei bspw. (batteriebetriebene) (Minimotor-) Pumpen die Flüssigkeit(en) aus dem/den Vorratsbehälter(n) 48 ansaugen und mit entsprechendem Druck durch die Leitung (en) 50 zu der oder den Nebeldüsen 142, 144 leiten können. Zudem kann, wie in Fig. 9 gezeigt, die Zuführeinheit 18 einen Sensor 152 aufweisen, der es erlaubt, zu bestimmen, ob die Materialbahn 12 gespannt ist. Der Sensor 152 kann mit der oder den Pumpen derart verbunden sein, dass die Pumpe(n) nur bei gespannter Materialbahn 12 die Flüssigkeit(en) aus dem/den Vorratsbehälter(n) 48 zu der oder den Nebeldüsen 142, 144 fördert/fördern. Ferner versteht es sich, dass die in Fig. 6a-9 gezeigten Merkmale auch in jeglicher anderer als der gezeigten Weise kombiniert werden können.

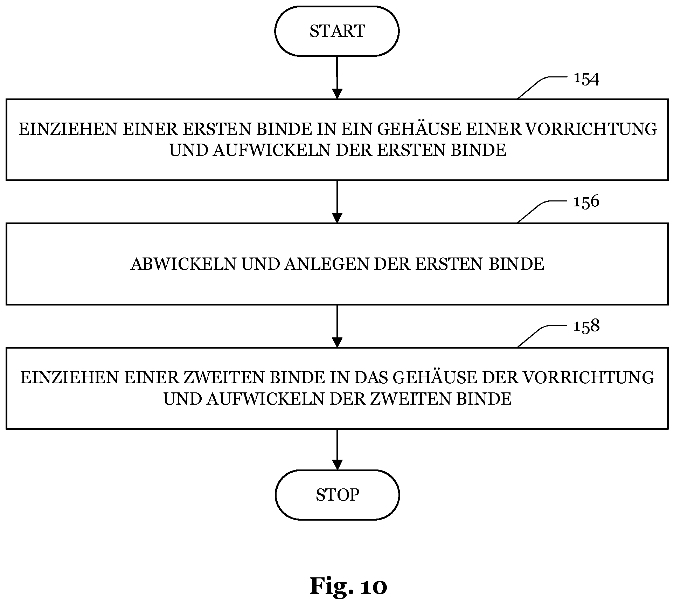

Fig. 10 zeigt ein Ablaufdiagramm eines Verfahrens zum Vorbereiten und Anlegen von Materialbahnen/Binden 12. Das Verfahren beginnt mit einem Schritt 154 des Einziehens einer ersten Materialbahn/Binde 12 in das Gehäuse 14, 22, 24 einer Vorrichtung 10, 26 und dem Aufwickeln der ersten Materialbahn/Binde 12. Nach einem Schritt 156 des Abwickelns und Anlegens der ersten Materialbahn/Binde 12, wird das Verfahren mit dem Schritt 158 des Einziehens einer zweiten Materialbahn/Binde 12 in das Gehäuse 14, 22, 24 der Vorrichtung 10, 26 und des Aufwickelns der zweiten Materialbahn/Binde 12 fortgesetzt.

### BEZUGSZEICHENLISTE

- 10: Vorrichtung
- 12: Materialbahn/Binde
- 12a: Materialbahnabschnitt
- 12b: Materialbahnabschnitt
- 14: Gehäuse
- 16: Wickeleinheit
- 18: Zuführeinheit
- 20a: Stoff (fest, flüssig, gasförmig)
- 20b: Stoff (fest, flüssig, gasförmig)
- 22: Gehäuse (Wickeleinheit)
- 24: Gehäuse (Zuführeinheit)
- 26: Vorrichtung
- 28: Halterung
- 30: Energiespeichereinheit
- 32: Motor
- 34: Steuereinheit
- 36: Kurbel
- 38: Bremse
- 40: Düse
- 42: Düse
- 44: Rolle
- 46: Gegendruckzylinder
- 48: Vorratsbehälter
- 50: Leitung
- 52: Anschluss
- 54: elastisches Element
- 56: elastische Wand
- 58: Unterteil
- 60: Oberteil
- 62: Scharniere
- 64: Federklammer
- 66: Lippe
- 68: Unterteil
- 70: Oberteil
- 72: Scharniere
- 74: Welle
- 76: Klammer
- 78: Leitseite
- 80: Leitseite
- 82: Querstab
- 84: Zahnhebel
- 86: Schenkelfeder
- 88: Zahnscheibe
- 90: Konstantkraftfeder
- 92: Steg
- 92a: Querstab
- 94: Spannrolle
- 96: Spannmutter
- 98: Schließhaken
- 100: Deckel
- 102: Kurbel
- 104: Exzenterachse
- 106: Desinfektionsmittel-Geber
- 108: Rolle
- 110: Kapillarkanal
- 112: Hahn
- 114: Aufnahme- und Verteilerrolle
- 114a: Einkerbung
- 116: Spenderbohrung
- 118: Klemmfeder
- 120: Spendergriff
- 122: Auffangtablett
- 124: Saugnapf
- 126: Bremsband
- 128: Leitflansch
- 130: Bremstrommel
- 132: Spannfeder
- 134: Federstift
- 136: Ankerhaken
- 138: Gewindestange
- 140: Spannmutter
- 142: Nebeldüse
- 144: Nebeldüse
- 146: Führungsfläche
- 148: Halterung
- 150: Öffnung
- 152: Sensor
- 154: Prozessschritt
- 156: Prozessschritt
- 158: Prozessschritt

## Patentansprüche

1. Vorrichtung (10), zur Handhabung von an einen Verwendungsort anzulegenden bandförmigen Materialbahnen (12), vorzugsweise Binden, umfassend:
ein Gehäuse (14, 22) mit einem in dem Gehäuse (14, 22) drehbar gelagerten Element einer Wickeleinheit (16), welche zum Einziehen einer Materialbahn (12) in das Gehäuse (14, 22) und zum Aufwickeln der Materialbahn (12) eingerichtet ist; und
eine der Wickeleinheit (16) vor- oder nachschaltbare Zuführeinheit (18) zum Aufbringen eines in einem Vorratsbehälter (48) bevorrateten festen, flüssigen oder gasförmigen Stoffes (20a, 20b) auf die Materialbahn (12).

2. Vorrichtung (10) nach Anspruch 1, wobei das Gehäuse (14, 22) von der der Wickeleinheit (16) vor- oder nachschaltbaren Zuführeinheit (18) abkoppelbar und vorzugsweise werkzeuglos abkoppelbar ist und vorzugsweise eine spaltförmige Öffnung zum Hindurchführen der Materialbahn (12) aufweist.

3. Vorrichtung (10) nach Anspruch 1 oder 2, wobei die Wickeleinheit (16) zum Abwickeln der Materialbahn (12) eingerichtet ist, und eine, einer beim Abwickeln aufzuwendenden Zugkraft entgegenwirkende, einstellbare Rückhaltekraft, Bremskraft oder Rückstellkraft, bereitstellt.

4. Vorrichtung (10) nach einem der Ansprüche 1 bis 3, wobei die der Wickelvorrichtung (16) vor- oder nachschaltbare Zuführeinheit (18) eingerichtet ist, den in dem Vorratsbehälter (48) bevorrateten festen, flüssigen oder gasförmigen Stoff (20a, 20b) beim Einziehen der Materialbahn (12) in das Gehäuse (14, 22) auf die Materialbahn (12) aufzubringen.

5. Vorrichtung (10) nach Anspruch 4, wobei die Vorrichtung (10) eingerichtet ist, den Massestrom des beim Einziehen der Materialbahn (12) in das Gehäuse (14, 22) auf die Materialbahn (12) aufgebrachten festen, flüssigen oder gasförmigen Stoffes (20a, 20b) in Abhängigkeit von der Einziehgeschwindigkeit zu steuern, wobei der Massestrom versiegt, wenn die Einziehgeschwindigkeit null ist.

6. Vorrichtung (10) nach einem der Ansprüche 1 bis 5, wobei ein Minimalwert der Einziehgeschwindigkeit einstellbar ist oder die Einziehgeschwindigkeit auf einen konstanten Wert einstellbar ist.

7. Vorrichtung (10) nach einem der Ansprüche 1 bis 6, wobei die Wickeleinheit (16) eine Energiespeichereinheit (30), vorzugsweise eine Konstantkraftfeder (90) aufweist, der durch Drehen des drehbar gelagerten Elements in eine Richtung Energie zugeführt und durch Drehen des drehbar gelagerten Elements in die andere Richtung Energie entnommen werden kann.

8. Vorrichtung (10) nach einem der Ansprüche 1 bis 7, wobei die vor- oder nachschaltbare Zuführeinheit (18) eine Spanneinrichtung, vorzugsweise eine Spannrolle (94), umfasst, die dazu eingerichtet ist, einen zwischen der Spanneinrichtung und dem im Gehäuse (14, 22) drehbar gelagerten Element geführten Materialbahnabschnitt (12b) beim Einziehen der Materialbahn (12) zu spannen.

9. Vorrichtung (10) nach einem der Ansprüche 1 bis 8, wobei die Wickeleinheit (16) eingerichtet ist, eine die Drehung des in dem Gehäuse (14, 22) drehbar gelagerten Elements verhindernde Sperrung aufzuheben, wenn auf einen durch das Gehäuse (14, 22) geführten und an dem Element befestigten Materialbahnabschnitt (12a, 12b) eine Zugspannung ausgeübt wird.

10. Vorrichtung (10) nach Anspruch 8 oder 9, wobei die vor- oder nachschaltbare Zuführeinheit (18) eine Rolle (44, 108, 114) umfasst, über die der Materialbahnabschnitt (12a) geführt wird, und die vor- oder nachschaltbare Zuführeinheit (18) eingerichtet ist, den festen, flüssigen oder gasförmigen Stoff (20a, 20b) mittels der Rolle (44, 108, 114) auf den Materialbahnabschnitt (12a) aufzubringen.

11. Vorrichtung (10) nach einem der Ansprüche 8 bis 10, wobei die vor- oder nachschaltbare Zuführeinheit (18) eine an den Vorratsbehälter (48) angeschlossene Düse (40, 42, 142, 144) aufweist, die eingerichtet ist, den festen, flüssigen oder gasförmigen Stoff (20a, 20b) direkt oder indirekt auf den Materialbahnabschnitt (12a) aufzubringen.

12. Vorrichtung (10) nach Anspruch 10, wobei der Vorratsbehälter (48) in der Rolle (44, 108, 114) angeordnet oder an die Rolle (44, 108, 114) angeschlossen ist und die vor- oder nachschaltbare Zuführeinheit (18) eingerichtet ist, den in dem Vorratsbehälter (48) bevorrateten festen, flüssigen oder gasförmigen Stoff (20a, 20b) über Öffnungen oder Aussparungen in der Rollenoberfläche auf den Materialbahnabschnitt (12a) aufzubringen.

13. Vorrichtung (26), zur Handhabung von Binden (12), umfassend:
ein Gehäuse (22) mit einem in dem Gehäuse (22) drehbar gelagerten Element einer Wickeleinheit (16), welches zum Einziehen einer Materialbahn (12) in das Gehäuse (22) und zum Aufwickeln der Materialbahn (12) eingerichtet ist,
wobei die Wickeleinheit (16) eingerichtet ist, eine die Drehung des in dem Gehäuse (22) drehbar gelagerten Elements verhindernde Sperrung aufzuheben, wenn auf einen durch das Gehäuse (22) geführten und an dem Element befestigten Materialbahnabschnitt (12a, 12b) eine Zugspannung ausgeübt wird, die oberhalb einer bestimmten Zugspannungsschwelle liegt.

14. Vorrichtung (26) nach Anspruch 13, wobei die Wickeleinheit (16) eine Energiespeichereinheit (30), vorzugsweise eine Konstantkraftfeder (90) aufweist, der durch Drehen des drehbar gelagerten Elements in eine Richtung Energie zugeführt und durch Drehen des drehbar gelagerten Elements in die andere Richtung Energie entnommen werden kann.

15. Verfahren zum Vorbereiten und Anlegen von Binden (12), umfassend:
Einziehen (154) einer ersten Binde (12) in das Gehäuse (14, 22) einer Vorrichtung (10, 26) nach einem der Ansprüche 1 bis 14 und Aufwickeln der ersten Binde (12);
Abwickeln (156) und Anlegen der ersten Binde (12);
Einziehen (158) einer zweiten Binde (12) in das Gehäuse (14, 22) der Vorrichtung (10, 26) und Aufwickeln der zweiten Binde (12); und
Abwickeln und Anlegen der zweiten Binde (12);
wobei beim Einziehen (158) oder Abgeben der Binden (12) vorzugsweise ein Desinfektions- und/oder ein heilförderndes Mittel auf die Binde (12) aufgebracht wird.

## Geänderte Patentansprüche

### Geänderte Patentansprüche gemäss Regel 137(2) EPÜ.

**1.** Vorrichtung (10), zur Handhabung von an einen Verwendungsort anzulegenden bandförmigen Materialbahnen (12), vorzugsweise Binden, umfassend:
ein Gehäuse (14, 22) mit einem in dem Gehäuse (14, 22) drehbar gelagerten Element einer Wickeleinheit (16), welche zum Einziehen einer Materialbahn (12) in das Gehäuse (14, 22) und zum Aufwickeln der Materialbahn (12) eingerichtet ist; und
eine der Wickeleinheit (16) vor- oder nachschaltbare Zuführeinheit (18) zum Aufbringen eines in einem Vorratsbehälter (48) bevorrateten festen, flüssigen oder gasförmigen Stoffes (20a, 20b) auf die Materialbahn (12).

**2.** Vorrichtung (10) nach Anspruch 1, wobei das Gehäuse (14, 22) von der der Wickeleinheit (16) vor- oder nachschaltbaren Zuführeinheit (18) abkoppelbar und vorzugsweise werkzeuglos abkoppelbar ist und vorzugsweise eine spaltförmige Öffnung zum Hindurchführen der Materialbahn (12) aufweist.

**3.** Vorrichtung (10) nach Anspruch 1 oder 2, wobei die Wickeleinheit (16) zum Abwickeln der Materialbahn (12) eingerichtet ist, und eine, einer beim Abwickeln aufzuwendenden Zugkraft entgegenwirkende, einstellbare Rückhaltekraft, Bremskraft oder Rückstellkraft, bereitstellt.

**4.** Vorrichtung (10) nach einem der Ansprüche 1 bis 3, wobei die der Wickelvorrichtung (16) vor- oder nachschaltbare Zuführeinheit (18) eingerichtet ist, den in dem Vorratsbehälter (48) bevorrateten festen, flüssigen oder gasförmigen Stoff (20a, 20b) beim Einziehen der Materialbahn (12) in das Gehäuse (14, 22) auf die Materialbahn (12) aufzubringen.

**5.** Vorrichtung (10) nach Anspruch 4, wobei die Vorrichtung (10) eingerichtet ist, den Massestrom des beim Einziehen der Materialbahn (12) in das Gehäuse (14, 22) auf die Materialbahn (12) aufgebrachten festen, flüssigen oder gasförmigen Stoffes (20a, 20b) in Abhängigkeit von der Einziehgeschwindigkeit zu steuern, wobei der Massestrom versiegt, wenn die Einziehgeschwindigkeit null ist.

**6.** Vorrichtung (10) nach einem der Ansprüche 1 bis 5, wobei ein Minimalwert der Einziehgeschwindigkeit einstellbar ist oder die Einziehgeschwindigkeit auf einen konstanten Wert einstellbar ist.

**7.** Vorrichtung (10) nach einem der Ansprüche 1 bis 6, wobei die Wickeleinheit (16) eine Energiespeichereinheit (30), vorzugsweise eine Konstantkraftfeder (90) aufweist, der durch Drehen des drehbar gelagerten Elements in eine Richtung Energie zugeführt und durch Drehen des drehbar gelagerten Elements in die andere Richtung Energie entnommen werden kann.

**8.** Vorrichtung (10) nach einem der Ansprüche 1 bis 7, wobei die vor- oder nachschaltbare Zuführeinheit (18) eine Spanneinrichtung, vorzugsweise eine Spannrolle (94), umfasst, die dazu eingerichtet ist, einen zwischen der Spanneinrichtung und dem im Gehäuse (14, 22) drehbar gelagerten Element geführten Materialbahnabschnitt (12b) beim Einziehen der Materialbahn (12) zu spannen.

**9.** Vorrichtung (10) nach einem der Ansprüche 1 bis 8, wobei die Wickeleinheit (16) eingerichtet ist, eine die Drehung des in dem Gehäuse (14, 22) drehbar gelagerten Elements verhindernde Sperrung aufzuheben, wenn auf einen durch das Gehäuse (14, 22) geführten und an dem Element befestigten Materialbahnabschnitt (12a, 12b) eine Zugspannung ausgeübt wird.

**10.** Vorrichtung (10) nach Anspruch 8 oder 9, wobei die vor- oder nachschaltbare Zuführeinheit (18) eine Rolle (44, 108, 114) umfasst, über die der Materialbahnabschnitt (12a) geführt wird, und die vor- oder nachschaltbare Zuführeinheit (18) eingerichtet ist, den festen, flüssigen oder gasförmigen Stoff (20a, 20b) mittels der Rolle (44, 108, 114) auf den Materialbahnabschnitt (12a) aufzubringen.

**11.** Vorrichtung (10) nach einem der Ansprüche 8 bis 10, wobei die vor- oder nachschaltbare Zuführeinheit (18) eine an den Vorratsbehälter (48) angeschlossene Düse (40, 42, 142, 144) aufweist, die eingerichtet ist, den festen, flüssigen oder gasförmigen Stoff (20a, 20b) direkt oder indirekt auf den Materialbahnabschnitt (12a) aufzubringen.

**12.** Vorrichtung (10) nach Anspruch 10, wobei der Vorratsbehälter (48) in der Rolle (44, 108, 114) angeordnet oder an die Rolle (44, 108, 114) angeschlossen ist und die vor- oder nachschaltbare Zuführeinheit (18) eingerichtet ist, den in dem Vorratsbehälter (48) bevorrateten festen, flüssigen oder gasförmigen Stoff (20a, 20b) über Öffnungen oder Aussparungen in der Rollenoberfläche auf den Materialbahnabschnitt (12a) aufzubringen.

**1.** Vorrichtung (10), zur Handhabung von an einen Verwendungsort anzulegenden bandförmigen Materialbahnen (12), vorzugsweise Binden, umfassend:
ein Gehäuse (14, 22) mit einem in dem Gehäuse (14, 22) drehbar gelagerten Element einer Wickeleinheit (16), welche zum Einziehen einer Materialbahn (12) in das Gehäuse (14, 22) und zum Aufwickeln der Materialbahn (12) eingerichtet ist; und
eine der Wickeleinheit (16) vor- oder nachschaltbare Zuführeinheit (18) zum Aufbringen eines in einem Vorratsbehälter (48) bevorrateten festen, flüssigen oder gasförmigen Stoffes (20a, 20b) auf die Materialbahn (12);
**dadurch gekennzeichnet, dass**
die Wickeleinheit (16) zum Abwickeln der Materialbahn (12) eingerichtet ist und eine, einer beim Abwickeln aufzuwendenden Zugkraft entgegenwirkende, einstellbare Rückhaltekraft, Bremskraft oder Rückstellkraft bereitstellt.

**2.** Vorrichtung (10) nach Anspruch 1, wobei das Gehäuse (14, 22) von der der Wickeleinheit (16) vor- oder nachschaltbaren Zuführeinheit (18) abkoppelbar und vorzugsweise werkzeuglos abkoppelbar ist und vorzugsweise eine spaltförmige Öffnung zum Hindurchführen der Materialbahn (12) aufweist.

**3.** Vorrichtung (10) nach Anspruch 1 oder 2, wobei die der Wickelvorrichtung (16) vor- oder nachschaltbare Zuführeinheit (18) eingerichtet ist, den in dem Vorratsbehälter (48) bevorrateten festen, flüssigen oder gasförmigen Stoff (20a, 20b) beim Einziehen der Materialbahn (12) in das Gehäuse (14, 22) auf die Materialbahn (12) aufzubringen.

**4.** Vorrichtung (10) nach Anspruch 3, wobei die Vorrichtung (10) eingerichtet ist, den Massestrom des beim Einziehen der Materialbahn (12) in das Gehäuse (14, 22) auf die Materialbahn (12) aufgebrachten festen, flüssigen oder gasförmigen Stoffes (20a, 20b) in Abhängigkeit von der Einziehgeschwindigkeit zu steuern, wobei der Massestrom versiegt, wenn die Einziehgeschwindigkeit null ist.

**5.** Vorrichtung (10) nach einem der Ansprüche 1 bis 4, wobei ein Minimalwert der Einziehgeschwindigkeit einstellbar ist oder die Einziehgeschwindigkeit auf einen konstanten Wert einstellbar ist.

**6.** Vorrichtung (10) nach einem der Ansprüche 1 bis 5, wobei die Wickeleinheit (16) eine Energiespeichereinheit (30), vorzugsweise eine Konstantkraftfeder (90) aufweist, der durch Drehen des drehbar gelagerten Elements in eine Richtung Energie zugeführt und durch Drehen des drehbar gelagerten Elements in die andere Richtung Energie entnommen werden kann.

**7.** Vorrichtung (10) nach einem der Ansprüche 1 bis 6, wobei die vor- oder nachschaltbare Zuführeinheit (18) eine Spanneinrichtung, vorzugsweise eine Spannrolle (94), umfasst, die dazu eingerichtet ist, einen zwischen der Spanneinrichtung und dem im Gehäuse (14, 22) drehbar gelagerten Element geführten Materialbahnabschnitt (12b) beim Einziehen der Materialbahn (12) zu spannen.

**8.** Vorrichtung (10) nach einem der Ansprüche 1 bis 7, wobei die Wickeleinheit (16) eingerichtet ist, eine die Drehung des in dem Gehäuse (14, 22) drehbar gelagerten Elements verhindernde Sperrung aufzuheben, wenn auf einen durch das Gehäuse (14, 22) geführten und an dem Element befestigten Materialbahnabschnitt (12a, 12b) eine Zugspannung ausgeübt wird.

**9.** Vorrichtung (10) nach einem der Ansprüche 1 bis 8, wobei die vor- oder nachschaltbare Zuführeinheit (18) eine Rolle (44, 108, 114) umfasst, über die der Materialbahnabschnitt (12a) geführt wird, und die vor- oder nachschaltbare Zuführeinheit (18) eingerichtet ist, den festen, flüssigen oder gasförmigen Stoff (20a, 20b) mittels der Rolle (44, 108, 114) auf den Materialbahnabschnitt (12a) aufzubringen.

**10.** Vorrichtung (10) nach Anspruch 7 bis 9, wobei die vor- oder nachschaltbare Zuführeinheit (18) eine an den Vorratsbehälter (48) angeschlossene Düse (40, 42, 142, 144) aufweist, die eingerichtet ist, den festen, flüssigen oder gasförmigen Stoff (20a, 20b) direkt oder indirekt auf den Materialbahnabschnitt (12a) aufzubringen.

**11.** Vorrichtung (10) nach Anspruch 9, wobei der Vorratsbehälter (48) in der Rolle (44, 108, 114) angeordnet oder an die Rolle (44, 108, 114) angeschlossen ist und die vor- oder nachschaltbare Zuführeinheit (18) eingerichtet ist, den in dem Vorratsbehälter (48) bevorrateten festen, flüssigen oder gasförmigen Stoff (20a, 20b) über Öffnungen oder Aussparungen in der Rollenoberfläche auf den Materialbahnabschnitt (12a) aufzubringen.
